(19) **Europäisches Patentamt / European Patent Office / Office européen des brevets**

(11) **EP 4 262 824 B1**

(12) **EUROPEAN PATENT SPECIFICATION**

(45) Date of publication and mention
of the grant of the patent:
**12.11.2025   Bulletin 2025/46**

(21) Application number: **21843776.2**

(22) Date of filing: **20.12.2021**

(51) International Patent Classification (IPC):
*A61K 31/734* (2006.01)   *A61K 31/695* (2006.01)
*A61K 31/715* (2006.01)   *A61K 47/36* (2006.01)
*A61P 1/04* (2006.01)     *A61K 31/7004* (2006.01)
*A61K 31/728* (2006.01)

(52) Cooperative Patent Classification (CPC):
(C-Sets available)
**A61P 1/04; A61K 9/0056; A61K 9/06;
A61K 31/695; A61K 31/7004; A61K 31/715;
A61K 31/728; A61K 31/731; A61K 31/734;
A61K 31/737; A61K 47/02**           (Cont.)

(86) International application number:
**PCT/IB2021/062042**

(87) International publication number:
**WO 2022/130359 (23.06.2022 Gazette 2022/25)**

(54) **MULTICOMPONENT COMPOSITION COMPRISING SUCROSE SULFATE, ALGINATE, GELLAN GUM FOR THE TREATMENT OF GASTRIC REFLUX**

MEHRSTOFFGEMISCH ENTHALTEND SACCHAROSESULFAT, ALGINAT, GELLANGUMMI ZUR BEHANDLUNG VON MAGENREFLUX

COMPOSITION À COMPOSANTS MULTIPLES COMPRENANT DU SULFATE DE SACCHAROSE, DE L'ALGINATE, DE LA GOMME GELLANE POUR LE TRAITEMENT DU REFLUX GASTRIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA**
Designated Validation States:
**MA**

(30) Priority:   **18.12.2020   IT 202000031409
                18.12.2020   IT 202000031421**

(43) Date of publication of application:
**25.10.2023   Bulletin 2023/43**

(73) Proprietor: **Drugs Minerals and Generics Italia
S.R.L.**
**In Forma Abbreviata D.M.G. Italia S.R.L.
00071 Pomezia (RM) (IT)**

(72) Inventors:
• **MERCURI, Luigi**
 **00071 Pomezia (RM) (IT)**
• **TIBERI, Licia**
 **00071 Pomezia (RM) (IT)**

(74) Representative: **Benedetto, Marco**
 **Marben S.r.l.**
 **Via Larga, 16**
 **20122 Milano (IT)**

(56) References cited:
 **WO-A1-2010/092468      WO-A1-2018/100468
 WO-A1-2019/159073      WO-A2-2019/207506
 GB-A- 2 349 570**

**(Cont. next page)**

• HAYAKAWA TAKUYA ET AL: "A thin layer of sucroseoctasulfate protects the oesophageal mucosal epithelium in reflux oesophagitis", SCIENTIFIC REPORTS, NATURE PUBLISHING GROUP, US, vol. 9, no. 1, 5 March 2019 (2019-03-05), pages 3559, XP009529708, ISSN: 2045-2322, DOI: 10.1038/S41598-019-39087-4

• SURDEA-BLAGA T ET AL: "Mucosal protective compounds in the treatment of gastroesophageal reflux disease. A position paper based on evidence of the Romanian society of neurogastroenterology", JOURNAL OF GASTROINTESTINAL AND LIVER DISEASES,, vol. 25, no. 4, 1 December 2016 (2016-12-01), pages 537 - 546, XP009529712, ISSN: 1841-8724, DOI: 10.15403/ JGLD.2014.1121.254.DEA

• ANONYMOUS: "KLENSKIN Sunscreen Spa Wash On", 4 December 2020 (2020-12-04), XP002804090, Retrieved from the Internet <URL:https://web.archive.org/web/ 20201204051700/https://shop.klenskin.com/ products/klenskin-spa> [retrieved on 20210902]

• ANONYMOUS: "Texture Spray", 24 October 2020 (2020-10-24), XP002804091, Retrieved from the Internet <URL:https://web.archive.org/web/ 20201024010835/https://sojournbeauty.com/ shop/texture/texture-spray/> [retrieved on 20210902]

(52) Cooperative Patent Classification (CPC): (Cont.)

C-Sets
A61K 31/695, A61K 2300/00;
A61K 31/695, C07C 233/18;
A61K 31/7004, C07C 233/18;
A61K 31/715, A61K 2300/00;
A61K 31/715, C07C 233/18;
A61K 31/728, A61K 2300/00;
A61K 31/728, C07C 233/18;
A61K 31/731, A61K 2300/00;
A61K 31/734, A61K 2300/00;
A61K 31/734, C07C 233/18;
A61K 31/737, A61K 2300/00

**EP 4 262 824 B1**

**Description**

The present invention relates to multicomponent compositions comprising sucrose sulfate or derivatives thereof, preferably sucrosofate (sucrose octasulfate) and/or sucralfate (sucrose sulfate complex and aluminium hydroxide), and to the use thereof for the treatment of gastric reflux and diseases or symptoms deriving therefrom. In particular, the present invention relates to a multicomponent composition comprising a gellan gum and an alginic acid or a relative salt thereof, such as for example a sodium, potassium alginate, calcium and magnesium alginate salt or relative mixtures thereof, for use in a method for the treatment of gastroesophageal reflux disease (GERD). Document GB2349570 discloses, in example 4, a composition comprising: (a) sodium alginate; (b) simethicone; (c) xanthan gum; (d) sodium bicarbonate for treating gastroesophageal reflux.

Parietal cells, located in the gastric mucosa and responsible for the secretion of hydrochloric acid, are capable of releasing H+ ions in the gastric lumen by exchanging them with K+ ions through the action of the H+/K+ pump. Chemical and physical stimuli induce the release of numerous mediators (gastrin, histamine, prostaglandins and somatostatin) which finely regulate this secretion. However, in some cases there is an imbalance of these regulations with resulting acid hypersecretion; which may lead to pathological conditions such as gastroesophageal reflux disease or to the formation of ulcers on the mucosa of the upper section of the digestive tract.

Parietal cells, located in the gastric mucosa and responsible for the secretion of hydrochloric acid, are capable of releasing H+ ions in the gastric lumen by exchanging them with K+ ions through the action of the $H^+/K^+$ pump. Chemical and physical stimuli induce the release of numerous mediators (gastrin, histamine, prostaglandins and somatostatin) which finely regulate this secretion. However, in some cases there is the imbalance in these regulations, resulting in acid hypersecretion. Said acid hypersecretion may lead to pathological conditions such as gastroesophageal reflux disease or pharyngolaryngeal (or extra-oesophageal) reflux disease or to the formation of ulcers on the mucosa of the upper section of the digestive tract.

Gastroesophageal reflux disease (GERD) is a clinical condition characterised by massive backflow of the gastro-duodenal content into the oesophagus. The highly acidic gastric content and bile contained therein in contact with the oesophageal mucosa lead to inflammatory conditions which may result in pyrosis or oesophageal ulcers. The characteristic symptoms of this disorder can be divided into typical (e.g. acid regurgitation, heartburn), atypical (e.g. feeling of gastric fullness, epigastric pain, dyspepsia, nausea) and extra-oesophageal (e.g. chronic cough, bronchospasm, inflamed throat, laryngitis).

Laryngopharyngeal reflux disease (LPRD) or extraoesophageal reflux disease is a clinical condition characterised by the backflow of the gastro-duodenal content from the stomach to the extraoesophageal regions such as the upper respiratory tract (the nasal cavity, the paranasal sinus, the oral cavity, the pharynx, the epiglottis and the larynx), the periocular region (conjunctiva and lacrimal apparatus) and the ear apparatus (Eustachian tube and middle ear).

Acid hypersecretion, gastroesophageal, laryngopharyngeal or extraoesophageal reflux and ulcers in gastric and oesophageal mucous membranes can be treated both by means of pharmacological therapy (H2 antihistamines; proton pump inhibitors, etc.), and by means of compounds which, in contact with the acidic environment or gastric mucosa, provide a protective action through physical mechanisms (for example a floating raft).

The technical problem addressed and solved by the present invention lies in providing innovative therapeutic solutions with anti-reflux action (treatment of gastric reflux and diseases and/or symptoms deriving therefrom) and highly effective gastroprotectant, without significant side effects, which can be used by a wide range of subjects in need, and cost-effective.

Following an extensive research and development program, the Applicant addresses and solves the aforementioned technical problem by providing an innovative composition, preferably for oral use, comprising a mixture M comprising or, alternatively, consisting of (a) alginic acid or a salt thereof (alginate), (b) a simethicone and/or dimethicone, (c) a plant polysaccharide selected from gellan gum, and (d) a sucrose sulfate or a derivative thereof (e.g. salt or complex), preferably sucrosofate or sucralfate, as described hereinafter in the present invention (in short, composition/s or mixture/s M of the invention).

The compositions or mixtures M of the present invention, according to any one of the described embodiments, exert an effective mechanical action for the treatment of gastric reflux, in order to reduce the diseases and/or symptoms related thereto. In addition, said compositions or mixtures M of the present invention exert an excellent muco-adhesive and muco-protecting action with ensuing ability to protect the oesophageal mucous membranes from the corrosive-inflammatory action of the gastric juices backflowing during the reflux episode, and the gastric mucosa (anti-acidity effect).

In particular, the compositions or mixtures M of the present invention, preferably for oral use, by virtue of the active components thereof and/or the synergy thereof are capable of: (i) increasing the quality, in terms of greater strength and greater elasticity, of the structure of the gel which is formed in the stomach starting from the compositions of the invention as a result of the acidic pH conditions (gel with floating raft effect/action), (ii) increasing the rapidity in the formation of said gel in the stomach, and/or (iii) exhibit a high gastroprotective or muco-protective capacity; and/or (iv) exhibit a high cicatrizing capacity.

As a matter fact, the Applicant surprisingly found that in an acidic pH environment such as the stomach, alginate gives rise to the formation of a floating raft gelatinous precipitate capable of forming a mechanical barrier which prevents the backflow of the gastric reflux at the level of the cardia and therefore to the oesophagus and the extra-oesophageal regions (for example, pharynx, larynx, upper respiratory tract, lacrimal duct).

In addition, when said (c) plant polysaccharide is gellan gum, this component allows to form, combined with alginate and the other components of the composition of the invention, a floating raft which is more resistant and long-lasting with respect to the ones known in the prior art. In particular, in the presence of cations gellan gum is capable of forming a gel. Given that salts are present in the gastric juice, gellan gum is capable of gelling *in situ* and, therefore, strengthening the volume of the raft.

Further, said high gastroprotective action or protective mucosa of the composition or mixture M of the invention is mainly, but not only, due to the ability thereof to form on the mucosa, for example the gastric mucosa, a highly effective muco-adhesive layer, in particular due to the presence - in the composition - of a sucrose sulfate or derivatives thereof, such as sucrosofate or sucralfate, as well as the plant polysaccharide (c), preferably gellan gum.

Advantageously, sucrosofate or sucralfate confer to the composition or mixture M of the invention a gastroprotective, cicatrizing and adhesiveness capacity to the mucosa, for example gastric and oesophageal mucosa, on which it forms a protective film.

Furthermore, sucrosofate or sucralfate confer to the composition or mixture M of the invention an antiinflammatory effect and an ability to maintain the integrity of the mucous membranes, preferably gastric and oesophageal mucosa.

With respect to sucralfate, sucrosofate has the advantage of not comprising aluminium and of having better palatability.

In addition, the compositions of the invention preferably do not comprise, entirely or almost entirely, sodium sources.

The compositions or mixtures of the present invention do not have significant side effects, they can be administered to a wide range of subjects in need, and they are easy and cost-effective to produce.

These and other objects which will be apparent from the detailed description that follows, are attained by the mixtures and by the composition of the present invention due to the technical characteristics claimed in the attached claims.

FIGURES

Figure 1 shows an example of the process for the preparation of the composition of the invention in an aqueous syrup formulation.

Figure 2 shows the formation of the floating raft according to the experimental conditions reported in experimental part.

Figures 3a and 3b right, relatively to gellan gum, show that on the paper-filter there was collected a compact gel and the filtrate was found to be entirely colourless, indicating that the instantaneous gelling of the polysaccharide did not allow the diffusion of the colouring molecule in the acidic solution.

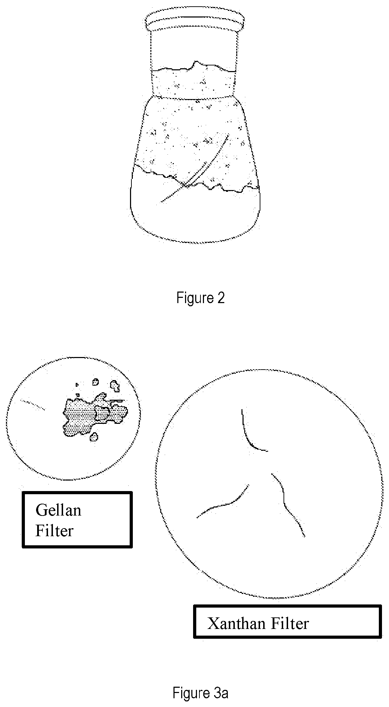

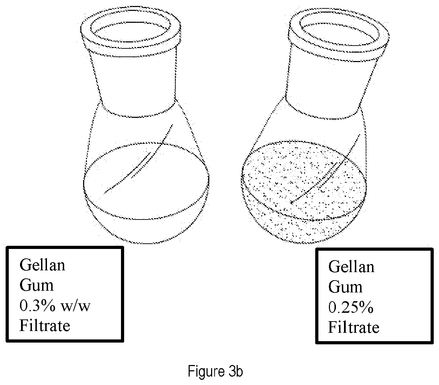

While Figures 3a and 3b left, relatively to xanthan gum, show that the paper-filter did not retain anything and the filtered solution was found to be coloured, given that no gel was formed and the colouring molecule is diffused in the acidic solution.

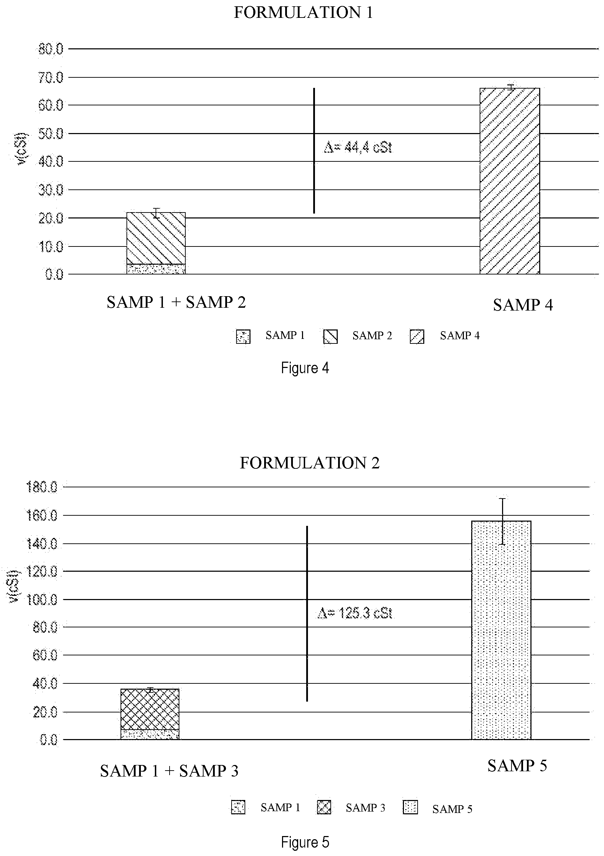

Figures 4 and 5 show that in Formulation 1 and in Formulation 2 (paragraph 3, data reported in Table 9) there is a significant increase in viscosity in the presence of mucins.

DETAILED DESCRIPTION OF THE INVENTION

Forming an object of the present invention is a composition comprising (i) a mixture M of active components comprising or, alternatively, consisting of:

- (a) an alginic acid or a pharmaceutical or food grade salt thereof, preferably an alkali metal or alkaline-earth metal alginate (e.g. magnesium, sodium, potassium and/or calcium) or an ammonium alginate (NH4(+));
- (b) a simethicone and/or dimethicone;
- (c) at least one plant polysaccharide, namely gellan gum;
- (d) sucrose octasulfate (referred to as as sucrosofate) or, alternatively, the sucrose octasulfate and poly aluminium hydroxide complex (referred to as sucralfate);

and, optionally, said composition comprises (ii) at least one pharmaceutical or food grade additive and/or excipient.

The mixture M of the composition of the present invention comprises (a), (b), (c), (d) and, optionally (f), according to any one of the described embodiments, it may further comprise (e) a bicarbonate salt and/or a carbonate salt with an alkali metal or alkaline-earth metal, preferably selected from sodium bicarbonate, potassium bicarbonate, magnesium bicarbonate, calcium bicarbonate, sodium bicarbonate, potassium carbonate, magnesium carbonate, calcium carbonate and mixtures thereof; preferably potassium bicarbonate.

An example of (e) potassium bicarbonate is potassium bicarbonate E501 (acidity regulator), for example EINECS No. 298-14-6 with Bulk density of about 0.99-1.12 $g/cm^3$ and Tapped density of about 1.14-1.25 $g/cm^3$.

Alginic acid is a natural linear polysaccharide, consisting of monomers of D-mannuronic and D-glucuronic acid linked by glycosidic $\beta(1\rightarrow4)$ bonds; structural formula $(C_6H_8O_6)_n$. In an acidic pH environment, such as in the stomach, the presence of numerous carboxyl groups along the chain causes alginate to form a low-density gel capable of floating and accumulating near the gastro-oesophageal sphincter; this behaviour leads to a decrease in the frequency and severity of episodes of gastroesophageal reflux. Given that alginic acid is not particularly soluble in water, an alginic acid salt, such as an alkali metal or alkaline-earth metal alginate, is preferably present in the composition of the invention.

At the acidic pH of the stomach, alginate precipitates in form of gel.

Advantageously, said(a) alginic acid or salt thereof, comprised in the composition or mixture M of the invention together with (b), (c), (d) and optionally (e) and/or (f) according to any one of the described embodiments, is or derives from an alginic acid having an average molecular weight comprised in the range from about 10 KDalton (KDa=1.000 Da) to about 600 KDa; preferably from about 100 KDa to about 400 kDa or 500 KDa, more preferably from 200 KDa to about 300 KDa, for example about 240 KDa (one Dalton (Da) equals to an atomic mass unit.

Said(a) alginic acid or salt thereof, comprised in the composition or mixture M of the invention together with (b), (c), (d) and, optionally, (e) and/or (f) (according to any one of the described embodiments), may be or derives from an alginic acid having an average molecular weight of about 200-300 KDa obtained from seaweed, such as for example, a magnesium alginate (e.g. CAS No. 37251-44-8) obtained from alginic acid with average molecular weight of about 230-250 KDa. Preferably, said alginic acid or alginate (a) does not comprise sodium.

Examples of (a) an alginic acid or alginate which can be used in the composition or mixture M of the invention together with (b), (c), (d) and, optionally, (e) and/or (f) (according to any one of the described embodiments), are represented by:

- Alginic acid EP (CAS No. 9005-32-7) in form of a straw yellow powder with an average particle size of about 80 mesh, a viscosity less than or equal to 50 cPs, an acidity index greater than or equal to 230, carboxyl groups of about 19-25, a pH of about 2-3.5;
- Calcium alginate (CAS No. 90005-35-0) with general formula $(C_6H_7Ca_{1/2}O6)_n$; structural units 195.16 (theoretical), 219 (average) and macromolecules: 10,000-600,000 (typical average);
- Magnesium alginate (CAS No. 37251-44-8) (composition: guluronic acid of about 65-75% and mannuronic acid of about 25-35% and a magnesium content of about 5.4-6.6) with a pH value of about 6-9.5, a viscosity (CPS) (LV, 12RPM) 7.5% natural basic solution 25°C of about 1,000-1,800;
- Potassium alginate, (CAS No. 90005-36-1), with general formula $(C_6H_7KO_6)n$; structural units 214.22 (theoretical), 238 (average) and macromolecules: 10,000-600,000 (typical average);
- Composition: sodium alginate E401 and sucrose, with a viscosity of about 550-750 mPas (1% sol.) and pH of about 6-8.5 (1% sol.);
- Sodium alginate, (CAS No. 90005-38-3), with general formula $(C_6H_7NaO_6)n$; structural units 198.11 (theoretical), 222 (average) and macromolecules: 10,000-600,000 (typical average).

The expression "simethicone" (example CAS 8050-81-5), alternatively referred to as activated dimethicone, refers to a polydimethylsiloxane (or dimethicone, example CAS 63148-62-9) activated with silica dioxide (synthetic polymer). Simethicone has a structural formula $(C_2H_6OSi)_n(SiO_2)_m$ with n and m variable (for example, n=6 and m=3).

Preferably, said active component (b), comprised in the composition or mixture M of the invention together with (a), (c), (d) and optionally (e) and/or (f) according to any one of the described embodiments, is simethicone, for example the compound with CAS No. 8050-81-5 referred to as $\alpha$-(trimethylsilyl)-$\omega$-methylpoly[oxy(dimethylsilylene)] in mixture with silica dioxide.

Examples of simethicone (b) which can be used in the compositions or mixtures M of the present invention, combined with (a), (b), (d) and, optionally (e) and/or (f) according to any one of the described embodiments, are represented by:

- simethicone in form of water-soluble simethicone (aqueous emulsion) with an antifoam activity of 15 seconds max.

and an active component content comprised (simethicone) from 29% to 32% by weight and a density of about 1 g/cm$^3$;

- simethicone such as a compound consisting of a fluid polydimethylsiloxane (PDMS) and an aerogel silica. Aerogel silica activates the antifoam properties of the fluid silicon. This compound has a specific gravity at 25°C of about 0.1; a viscosity at 25°C of about 3000 cP; an efficiency less than 15 seconds; a composition of PDMS of about 92.5-97.5% and silica of about 4-7%;
- simethicone in form of an emulsion with a solid content of about 31-34% by weight; an active ingredient ("Simethicone", USP) of about 30% by weight, a density at 25°C of about 1 g/ml, a viscosity at 25°C of about 1500-5000 mPas, a pH at 25°C of about 3-5.

Alternatively, said active component (b), comprised in the composition or mixture M of the invention together with (a), (c), (d) and, optionally, (e) and/or (f) (according to any one of the described embodiments), may be dimethicone.

Examples of dimethicone (b) which can be used in the compositions or mixtures M of the present invention, combined with (a), (b), (d) and, optionally, (e) and/or (f) (according to any one of the described embodiments), are represented by:

- dimethicone CAS No. 63148-62-9, in liquid form with a kinematic viscosity at 25°C of about 332.5-367.5 mm2/s, a density at 25°C of about 0.9660-0.9720 g/ml, a refractive index at 25°C of about 1.4025-1.4045, chemical name polydimethylsiloxane;
- dimethicone CAS No. 63148-62-9, in liquid form with a refractive index of about 1.4030-1.4050, a density at 25°C of about 0.95-0.97 g/ml and a viscosity at 25°C of about 332-368 mm2/ s;
- PEG-14 Dimethicone (components: PEG-14 Dimethicone 95% by weight and propylene glycol 5% by weight) CAS No. 68937-54-2, in liquid form with a refractive index of about 1.4500-1.4600, a density at 25°C of about 1.063-1.077 g/ml and a viscosity at 25°C of about 300-600 mPas;
- Dimethicone (component: *Stearoxy Dimethicone* 100%) CAS No. 68554-53-0, with a refractive index of about 1.4210-1.4270, a density at 50°C of about 0.8600-0.9000 g/ml and a recrystallisation of about 20-30 °C.

Further examples of dimethicone which can be used in the context of the present invention are: cetyl dimethicone, stearyl dimethicone, stearoxy dimethicone, behenoxy dimethicone and all polymers belonging to the group of dimethicone copolyols such as for example: dimethicone PEG-8 adipate, dimethicone PEG-8 benzoate, dimethicone PEG-7 phosphate, dimethicone PEG-10 phosphate, dimethicone PEG/PPG-20/23 benzoate, PEG/PG-7 dimethicone, PEG-10 dimethicone, PEG-9 dimethicone, PEG-10 dimethicone, PEG-12 dimethicone, PEG-3 dimethicone, PEG-7 dimethicone, PEG-14 dimethicone, PEG/PPG-8/14 dimethicone, PEG/PPG-3/10 dimethicone, PEG/PPG-4/12 dimethicone, PEG/PPG-6/11 dimethicone, PEG/PPG-4 dimethicone, PEG/PPG-15/15 dimethicone, PEG/PPG-16/2 dimethicone, PEG/PPG-17/18 dimethicone, PEG/PPG-18/18 dimethicone, PEG/PPG-19/ PPG-20/6 dimethicone, PEG/PPG-20/15 dimethicone, PEG/PPG-20/20 dimethicone, PEG/PPG-20/23 dimethicone, PEG/PPG-20/29 dimethicone, PEG/PPG-22/23 dimethicone, PEG/PPG-22/24 dimethicone, PEG/PPG-23/6 dimethicone, PEG/PPG-25/25 dimethicone and PEG/PPG-27/27 dimethicone.

Simethicones and dimethicones are inert and water-repellent silicone compounds capable of facilitating gastric emptying.

Gellan gum (c.i) is a linear polysaccharide composed of tetrasaccharide units formed by glucose-rhamnose-glucose-glucuronic acid units.

Advantageously, said(c.i) gellan gum, comprised in the composition or mixture M of the invention together with (a), (b), (d) and, optionally, (e) and/or (f), has an average molecular weight comprised in the range from 50 kDa to about 2000 kDa; preferably from about 100 kDa to about 600 kDa, more preferably from about 200 kDa to about 300 kDa. An example of gellan gum (c.i) which can be used in the context of the present invention together with (a), (b), (c) and, optionally, (e) and/or (f), is the gellan gum having CAS No. 71010-52-1 (example of product under the trade name: Gellan Gum Kelcogel CG-LA).

Xanthan gum (c.ii), or simply xanthan, is a polysaccharide having molecular formula (monomer) $C_{35}H_{49}O_{29}$, for example CAS No. 11138-66-2, for food use EEC No. E415.

Guar gum (c.iii) is a polysaccharide whose main constituent is a galactomannan, a trisaccharide consisting of mannose and galactose units, specifically polymerised to form α-D-mannopyranosyl chains linked with a β-D-(1-4) glycosidic bond and with a molecular weight of around 200-300 kDa or higher, to form a 1-4 straight chain with short 1-6 side branches of galactose. Molecular formula C18H30O15, for example CAS No. 9000-30-0, for food use EEC No. E412.

Carrageenans are (c.iv) are a family of linear sulfated polysaccharides. All carrageenans are high molecular weight polysaccharides composed of repeating units of galactose and 3,6 anhydrogalactose (3,6-AG), both sulfated and non-sulfated. The units are linked by alternating α-1,3 and β-1,4 glycosidic bonds.

The hyaluronic acid or the salt thereof (c.v) (for example of sodium or potassium) which can be used in the context of the present invention may be linear or crosslinked and have a weighted average molecular weight comprised from 500 kDa to 2500 kDa (kDa abbreviation of kilodalton), for example 600 kDa, 800 kDa, 1000 kDa, 1200 kDa, 1400 kDa, 1600 kDa, 1800 kDa, 2000 kDa or 2200 kDa (for example CAS No. 9004-61-9).

The chondroitin sulfate or the salt thereof (c.vi) (for example of sodium or potassium) which can be used in the context of the present invention may have a weighted average molecular weight comprised from 50 kDa a 2000 kDa, for example 100 kDa, 200 kDa, 400 kDa, 600 kDa, 800 kDa, 1000 kDa, 1200 kDa, 1400 kDa, 1600 kDa, or 1800 kDa. Said chondroitin sulfate which can be used in the context of the present invention may be obtained from extracts of animal cartilage, mainly bovine and pig tissues (for example: trachea, ear and nose) or chicken tissues, or from shark, fish (for example rajiformes) or volatile cartilage. The mixture M of the composition of the invention may comprise or, alternatively, consist of: (a) alginic acid or a salt thereof, such as an alkali metal or alkaline-earth metal alginate selected from: magnesium alginate, sodium alginate, potassium alginate, calcium alginate and a mixture thereof; preferably magnesium alginate; (b) a simethicone and/or dimethicone; preferably simethicone; (c) at least one plant polysaccharide namely gellan gum; (d) sucrosofate or sucralfate; and optionally (e) and/or (f).

Said component (c) at least one plant polysaccharide, comprised in the mixture of the invention together with (a), (b), (d) and, optionally, (e) and/or (f), may be a mixture of said specific plant polysaccharides, according to the claims, for example gellan gum and xanthan gum, or gellan gum and guar gum, or gellan gum and carrageenan, or gellan gum and hyaluronic acid, or gellan gum and chondroitin sulfate.

Preferably, the mixture M of the composition of the invention comprises or, alternatively, consists of: (a) magnesium alginate; (b) a simethicone and/or dimethicone; preferably simethicone; (c) at least one plant polysaccharide, namely gellan gum; (d) sucrosofate or sucralfate; and optionally (e) and/or (f).

In a preferred embodiment, the mixture M of the composition of the invention comprises or, alternatively, consists of: (a) alginic acid or a salt thereof, such as an alkali metal or alkaline-earth metal alginate selected from: magnesium alginate, sodium alginate, potassium alginate, calcium alginate and a mixture thereof; preferably magnesium alginate; (b) a simethicone and/or dimethicone; preferably simethicone; (c) a gellan gum; and (d) sucrosofate or sucralfate; and optionally (e) and/or (f).

According to a preferred example of the composition of the invention, themixture M comprises or, alternatively, consists of: (a) magnesium alginate;
(b) a simethicone and/or dimethicone; preferably simethicone; (c) gellan gum; and (d) sucrosofate or sucralfate; and optionally (e) and/or (f).

Regarding the compositions of the present invention, it was analysed and observed that, in the acidic pH environment i similar to the stomach pH, gellan gum is capable of gelling therefore contributing - together with the alginate salt to the formation of a liquid gel, referred to as floating raft when this gel is formed in the stomach of the subject treated with the composition of the invention.

"Liquid gels" show a behaviour similar to that of strong gels when a slow and weak shear strength is imparted thereto, while they have a behaviour similar to that of viscous liquids if the imparted shear strength is strong and fast. The behaviour of liquid gels can be exploited to prevent the sedimentation of particles in a suspension.

The gelling of gellan gum in an acidic pH environment is due to the polysaccharide passing from the random coil conformation to a double helix conformation; the double helices, formed in a neutral environment and with low ionic strength, tend to repel due to the negatively charged carboxyl groups; by lowering the pH of the environment and increasing the ionic strength thereof, these repulsions are absent, thus allowing the formation of junction zones between the polysaccharide chains and the resulting formation of a three-dimensional network.

Given the chemical characteristics of gellan gum, it can be assumed that in the acidic pH stomach environment, gellan gelling together with alginate is facilitated both by the formation of junction zones between the double helices and by the stabilisation thereof due to the ionic interaction of the latter with the cation ions (e.g. of alkali metal or alkaline-earth metal) released by the alginate salt. Therefore, in the acidic pH environment of the stomach, the composition of the invention is capable of forming a floating raft which is more resistant and stable with respect to the products currently known in the art, due to the interaction and/or synergy of gellan gum and alginate.

In addition, gellan gum confers a marked mucoadhesive capacity to the composition or mixture M of the invention.

In the context of the present invention, the expression "derivative" of a sucrose sulfate is preferably used to indicate a sucrose sulfate salt or a sucrose sulfate complex, preferably a sucrose octasulfate salt or complex.

A sucrose sulfate salt preferably comprised in the mixture M of the composition of the invention, together with the components (a), (b) and (c) and, optionally, (e) and/or (f) (according to any one of the described embodiments), is sucrose octasulfate, alternatively referred to as sucrosofate, or an alkali metal or alkaline-earth metal salt thereof, preferably sucrose octasulfate potassium salt (for example CAS No. 76578-81-9) or sucrose octasulfate sodium salt (for example CAS No. 74135-10-7).

Alternatively, a sucrose octasulfate complex preferably comprised in the mixture M of the composition of the invention, together with the components (a), (b), (c) and, optionally, (e) and/or (f) according to any one of the described embodiments, is the sucrose octasulfate-poly aluminium hydroxide complex, alternatively referred to as sucralfate.

Sucrose octasulfate or sucrosofate (in short SOS), has IUPAC name [(2R,3R,4S,5R,6R)-2-[(2S,3S,4R,5R)-3,4-disulfooxy-2,5-bis(sulfooxymethyl)oxolan-2-yl]oxy-4,5-disulfooxy-6-(sulfooxymeth yl)oxan-3-yl] hydrogen sulfate, molecular formula $C_{12}H_{22}O_{35}S_8$, and for example CAS No. 57680-56-5.

In the context of the present invention, the expression "sucrosofate" is used to indicate both sucrose octasulfate and the alkali metal or alkaline-earth meta thereof (e.g. potassium sucrosofate or sodium sucrosofate).

Examples of sucrosofate which can be used in the context of the present invention, combined with (a), (b), (c) and, optionally, (e) and/or (f) (according to any one of the described embodiments), are sucrosofate potassium heptahydrate salt (molecular formula C12H14O35S8 8K 7H2O) and sucrosofate potassium anhydrous salt (molecular formula C12H14O35S8 8K).

The mixture M of the composition of the invention may comprise or, alternatively, consist of: (a) an alginic acid or a salt thereof, such as an alkali metal or alkaline-earth metal alginate selected from: magnesium alginate, potassium alginate, calcium alginate, sodium alginate, and a mixture thereof; preferably magnesium alginate, advantageously derive from an alginic acid having an average molecular weight of about 200-300 kDa; (b) a simethicone and/or dimethicone; preferably simethicone (for example CAS No. 8050-81-5); (c) at least one plant polysaccharide, namely gellan gum; and (d) sucrosofate; and optionally (e) and/or (f)

Preferably, the mixture M of the composition of the invention comprises or, alternatively, consists of: (a) magnesium alginate, preferably having an average molecular weight of about 200-300 kDa (for example CAS No. 37251-44-8); (b) a simethicone and/or dimethicone; preferably simethicone (for example CAS No. 8050-81-5); (c) a plant polysaccharide selected from: gellan gum, xanthan gum, guar gum, carrageenan, hyaluronic acid or a salt thereof, chondroitin sulfate or a salt thereof; preferably gellan gum (for example CAS No. 71010-52-1); and (d) sucrosofate; and optionally (e) and/or (f).

Even more preferably, the mixture M of the composition of the invention may comprise or, alternatively, consist of: (a) an alginic acid or a salt thereof, such as an alkali metal or alkaline-earth metal alginate selected from: magnesium alginate, potassium alginate, calcium alginate and a mixture thereof; preferably magnesium alginate, preferably having an average molecular weight of about 200-300 kDa (for example CAS No. 37251-44-8); (b) a simethicone and/or dimethicone; preferably simethicone (for example CAS No. 8050-81-5); (c) a gellan gum (for example CAS No. 71010-52-1); and (d) sucrosofate; and optionally (e) and/or (f).

The sucrose octasulfate and poly aluminium hydroxide complex or sucralfate has molecular formula C12H54A116O75S8, molecular mass (u) 2 086.75 g/mol, and for example CAS No. 54182-58-0.

The mixture M of the composition of the invention may comprise or, alternatively, consist of: (a) an alginic acid or a salt thereof, such as an alkali metal or alkaline-earth metal alginate selected from: magnesium alginate, potassium alginate, calcium alginate, sodium alginate, and a mixture thereof; preferably magnesium alginate, advantageously derive from an alginic acid having an average molecular weight of about 200-300 kDa; (b) a simethicone and/or dimethicone; preferably simethicone (for example CAS No. 8050-81-5); (c) a plant polysaccharide, namely gellan gum (for example CAS No. 71010-52-1); and (d) sucralfate; and optionally (e) and/or (f).

Preferably, the mixture M of the composition of the invention comprises or, alternatively, consists of: (a) magnesium alginate, preferably having an average molecular weight of about 200-300 kDa (for example CAS No. 37251-44-8); (b) a simethicone and/or dimethicone; preferably simethicone (for example CAS No. 8050-81-5); (c) gellan gum (for example CAS No. 71010-52-1); and (d) sucralfate; and optionally (e) and/or (f).

According to an example of the composition of the invention, the mixture M comprises or, alternatively, consists of: (a) an alginic acid or a salt thereof, such as an alkali metal or alkaline-earth metal alginate selected from: magnesium alginate, potassium alginate, calcium alginate, sodium alginate and a mixture thereof; preferably magnesium alginate, preferably having an average molecular weight of about 200-300 kDa (for example CAS No. 37251-44-8); (b) a simethicone and/or dimethicone; preferably simethicone (for example CAS No. 8050-81-5); (c) a gellan gum (for example CAS No. 71010-52-1); and (d) sucralfate; and optionally (e) and/or (f).

The composition or mixture M of the present invention comprising (a), (b), (c) (preferably (c.i) gellan gum), (d) and, optionally, (e) (according to any one of the described embodiments) may further comprise (f) an alkali metal or alkaline-earth metal salt, preferably wherein said alkali metal or alkaline-earth metal is the same of the component (a) when said (a) is in form of alginate salt. Said (f) alkali metal or alkaline-earth metal salt may be selected from magnesium salt, potassium salt, calcium salt or a mixture thereof, for example magnesium chloride, potassium chloride, calcium chloride; preferably magnesium chloride. Preferably (f) does not comprise sodium.

The presence - in the composition of the invention - of a given amount of the component (f) alkali metal or alkaline-earth metal salt, for example a magnesium salt such as magnesium chloride, facilitates the formation of liquid gel in particular when the composition is formulated in aqueous-based liquid form (e.g. syrup).

Based on the chemical characteristics of gellan gum, it was assumed that in acidic environment the gelling of gellan gum is facilitated both by the formation of junction zones between the double helices and by the stabilisation thereof due to the ionic interaction of the latter, in acidic environment, with the alkali metal or alkaline-earth metal released by the alginate (a) and, if present, by the alkali metal or alkaline-earth metal salt (f).

Advantageously, the mixture M of the composition of the invention comprises or, alternatively, consists of: (a) an alginic acid or a salt thereof, such as an alkali metal or alkaline-earth metal alginate selected from: magnesium alginate, potassium alginate, calcium alginate, sodium alginate and a mixture thereof; preferably magnesium alginate; (b) a simethicone and/or dimethicone; preferably simethicone; (c) gellan gum; (d) sucrosofate or sucralfate; preferably sucrosofate; (e) an alkali metal or alkaline-earth metal bicarbonate or carbonate; preferably potassium bicarbonate; and (f) an alkali metal or alkaline-earth metal salt; preferably magnesium chloride.

According to an example of the composition of the invention, the mixture M comprises or, alternatively, consists of: (a) magnesium alginate; (b) simethicone; (c) gellan gum (d) sucrosofate; (e) potassium bicarbonate; (f) magnesium chloride, and optionally additives and/or excipients.

According to a further example of the composition of the invention, the mixture M comprises or, alternatively, consists of: (a) magnesium alginate; (b) simethicone; (c) gellan gum or xanthan gum; (d) sucralfate; (e) potassium bicarbonate; (f) magnesium chloride, and optionally additives and/or excipients.

The compositions of the present invention, comprising said (i) mixture M comprising (a), (b), (c), (d) and, optionally, (e) and/or (f), may comprise (ii) at least one acceptable pharmaceutical or food grade additive and/or excipient, that is a substance devoid of therapeutic activity suitable for pharmaceutical or food use. In the context of the present invention, the acceptable additives and/or excipients for pharmaceutical or food use comprise all the ancillary substances known to the person skilled in the art for the preparation of compositions in solid, semi-solid or liquid form, such as for example, diluents, solvents (including water), solubilisers, acidifiers, thickeners, sweeteners, flavour enhancement agents, dyes, sweeteners, lubricants, surfactants, preservatives, stabilisers, pH stabilising buffers and mixtures thereof.

Preferably, the composition or the mixture M of the present invention M does not comprise a histamine 2 receptor antagonist (H2 antagonist), such as for example ranitidine, famotidine or cimetidine.

The compositions or mixtures M subject of the present invention, according to any one of the described forms, may be in a liquid form, such as solution, dispersion or suspension of a solid in a liquid, in semi-solid form, such as gel, cream or foam, or in solid form, such as powder, granules, microgranules, flakes, tablets or capsules.

Preferably, the compositions or mixtures M of the invention are formulated in a form suitable for administration through the oral route, for example in liquid form or in solid form.

The compositions or mixtures M of the invention may be formulated in solid form, preferably in form of granules, powder or flakes for oral suspension or dispersion on aqueous base (aqueous formulation). For example, the composition of the invention in said solid form is administered to the treated subjects in an aqueous formulation by dispersing or suspending the composition of the invention - solid in aqueous medium or water - before administration.

Alternatively, the composition of the invention is preferably formulated in aqueous-based liquid form (aqueous formulation), for example in form of suspension or dispersion such as a water-based syrup.

The composition of the invention, when in an aqueous formulation (for example in form of solid dispersed or suspended in an aqueous medium, or in form of syrup or the like) shows high stability (>= 30 months), homogeneity and absence of formation of precipitate.

Said aqueous formulation of the composition of the invention, such as a suspension of a solid in aqueous medium or a syrup, shows thixotropic characteristics, which make it significantly with greater compliance for the treated subject. Furthermore, during deglutition by the treated subject, the composition of the invention adheres - highly effectively - to the pharyngeal and oesophageal mucosa resulting in greater attenuation of laryngopharyngeal and oesophageal irritation events caused by gastric reflux. Advantageously, the composition of the present invention in aqueous based liquid form (for example syrup) comprises active components in the following percentages by weight with respect to the total weight of the composition:

- water in a percentage comprised in the range from 40% to 90%; preferably from 55% to 80%, more preferably from 60% to 75%;
- said (a) alginic acid or a salt thereof, preferably magnesium alginate, in a percentage comprised in the range from 0.5% to 15% (for example 1%, 2%, 3%, 4%, 5%, 8%, 10%, 12%, or 14%); preferably from 1% a 10%, more preferably from 1% to 5% (for example about 2.5% of magnesium alginate, such as CAS No. 37251-44-8);
- said (b) simethicone and/or dimethicone, preferably simethicone, in a percentage comprised in the range from 0.05% to 15% (for example 1%, 2%, 3%, 4%, 5%, 6%, 7%, 8%, or 9%); preferably from 0.1% to 10%, more preferably from 0.5% to 5% (for example about 1.5% of simethicone, such as CAS No. 8050-81-5);
- said (c) gellan gum in a percentage comprised in the range from 0.01% to 20%, preferably from 0.05% to 15%, preferably from 0.05% to 10% (for example 0.1%, 0.5%, 1%, 2%, 3%, 4%, 6%, 7%, 8%, 9% or 10%); preferably from 0.1% to 5%, more preferably from 0.1% to 2% (for example about 0.2% or 0.3% or 0.4% or 0.5% of gellan gum, such as CAS No. 71010-52-1);
- said (d) sucrosofate or sucralfate, preferably sucrosofate in a percentage comprised in the range from 0.1% to 20% (for example, 0.5%, 1%, 1.5%, 2%, 2.5%, 3%, 3.5%, 4%, 4.5%, 5%, 6%, 7%, 8%, 9%, 10%, 12%, 14%, 16%, or 18%); preferably from 0.1% to 5%, more preferably from 0.1% to 5%; and, optionally,
- said (e) bicarbonate and/or carbonate salt of an alkali metal or of an alkaline earth metal, if present, in a percentage comprised in the range from 0.1% to 10% (for example 1%, 2%, 3%, 4%, 5%, 6%, 7%, 8%, or 9%); preferably from 0.5% to 5%, more preferably from 0.5% to 2.5% (for example about 1% of potassium bicarbonate).

Preferably, in the mixture M of the composition of the invention the by weight ratio between the components (a) alginate (*e.g.* magnesium alginate) and (b) simethicone and (c.i) gellan gum ((a):(b):(c.i)) is comprised in the range from about 10:10:5 to about 10:2:1, preferably from about 10:8:5 to 10:3:1, for example about 10:5,6:1,3.

In the context of the present invention, the expression "composition" is used to indicate a pharmaceutical composition and/or a medical device composition and/or dietary supplement or dietary supplement composition or foodstuff.

Forming an object of the present invention are the compositions and mixtures M of the present invention (comprising (a), (b), (c), (d) and, optionally, (e) and/or (f), according to any one of the described embodiments) for use as medicament.

Forming an object of the present invention are the compositions and mixtures M of the present invention (comprising (a), (b), (c), (d) and, optionally, (e) and/or (f), according to any one of the described embodiments) for use in a method for the preventive and/or curative treatment of gastric reflux or gastroesophageal reflux disease or laryngopharyngeal reflux or extra-oesophageal reflux disease, and/or a disease, disorder or symptom associated with said gastric reflux in a subject in need, wherein said treatment method provides for the administration of a therapeutically effective dose of one of said compositions or mixtures M subject of the present invention to said subject.

Said diseases, disorders or symptoms associated with gastric reflux are selected from: laryngopharyngeal or extra-oesophageal reflux disease, oesophagitis (acute or chronic inflammation of the oesophagus mucosa), oesophageal ulcers, de-epithelialization of the oesophageal mucosa, acid regurgitation, heartburn, feeling of gastric fullness, epigastric pain, dyspepsia, nausea, chronic cough, bronchospasm, inflamed throat, laryngitis, globus sensation or hypopharyngeal bolus, pyrosis, dysphonia, nasopharyngeal inflammation, and all disorders primarily caused or co-caused by gastric reflux.

Said diseases, disorders or symptoms associated with gastric reflux may be present even in the absence of a diagnosis of a gastroesophageal reflux disease.

Furthermore, the compositions and mixtures M of the present invention, comprising (a), (b), (c), (d) and optionally (e) and/or (f) according to any one of the described embodiments, are for use in the treatment of re-epithelization, as a single treatment or as an adjuvant of further treatments.

Forming an object of the present invention are a method for the preventive and/or curative treatment of gastric reflux or gastroesophageal reflux disease or laryngopharyngeal or extra-oesophageal reflux disease, and/or of a disease or symptom associated with said gastric reflux in a subject in need, wherein said treatment method provides for the administration - to said subject - of a therapeutically effective dose of one of said compositions or mixtures M subject of the present invention (comprising (a), (b), (c), (d) and optionally (e) and/or (f), according to any one of the described embodiments).

Forming an object of the present invention are the non-therapeutic use of the compositions and mixtures M of the present invention (comprising (a), (b), (c), (d) and optionally (e) and/or (f), according to any one of the described embodiments) for gastric or gastroesophageal or laryngopharyngeal or extra-oesophageal disorders in a healthy subject, wherein said use provides for the administration of one of said compositions and mixtures M of the present invention to said subject.

Forming an object of the present invention is a process for the preparation of the composition of the present invention formulated in aqueous based liquid form (e.g. syrup), wherein said process comprises the steps of:

1. preparing the total amount of water prescribed and dividing it into a first aliquot (aliquot 1), equal to about 20%-60% v/v (for example 25%, 35%, 45%, 50%, or 55%), preferably 30%-40% v/v, with respect to the total water volume, and into a second aliquot (aliquot 2) for the remaining part; followed by

2. adding to said water aliquot 2 said (e) alkali metal or alkaline-earth metal bicarbonate and/or carbonate salt (*e.g.* potassium bicarbonate); followed by

3. further adding to said water aliquot 2 (a) alginic acid or a salt thereof (e.g. magnesium alginate), preferably (b) simethicone and/or dimethicone (e.g. simethicone or simethicone CAS No. 8050-81-5), and the remaining raw materials (e.g. alkali metal or alkaline-earth metal bicarbonate or carbonate, preferably potassium bicarbonate), except for (c) a polysaccharide (for example gellan gum) and, if present, said (f) alkali metal or alkaline-earth metal salt (*e.g.* magnesium chloride), and leaving under stirring to obtain a mixture A; in this step (3), (d) sucrosofate or sucralfate may also be added to said aliquot 2; followed by or simultaneously with said steps 2-3;

4. heating the water aliquot 1 to the temperature of 60°C-90°C, preferably 70°C-80°C, more preferably about 75°C, adding (c) gellan gum (*e.g.* gellan gum CAS No. 71010-52-1) and leaving under stirring to obtain a suspension B after step 4; in this step (4), (d) sucrosofate or sucralfate may also be added to said aliquot 1; optionally followed by the step 5 of

5. adding said (e) alkali metal or alkaline-earth metal salt (*e.g.* magnesium chloride) and leaving under stirring for a few minutes to obtain a suspension B after step 5; step 4 or step 5 followed by

6. combining said suspension B after step 4 or after step 5 with the mixture A obtained from step 3 to obtain the suspension C; followed by

7. stirring the suspension C up to obtaining a homogeneous appearance, subsequently allowing to cool obtaining a compact and weak gel; followed by

8. placing the gel obtained in step 7 under stirring again and obtaining a viscous and homogeneous suspension.

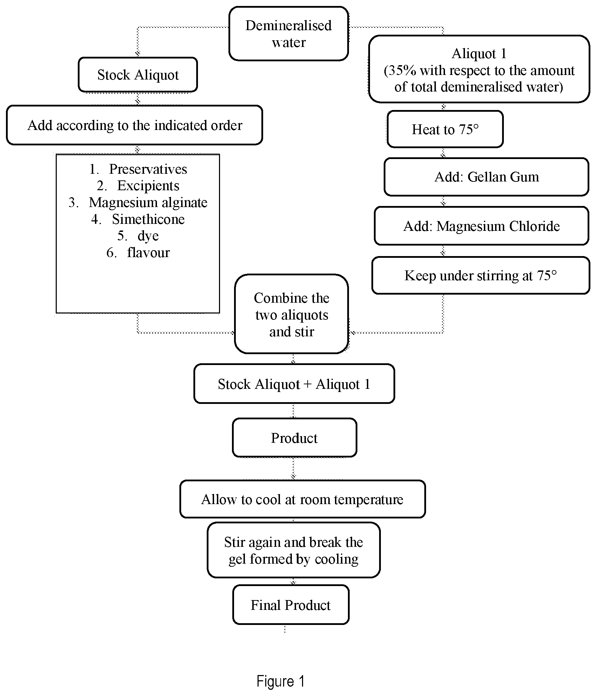

An example of the process for the preparation of the composition of the invention in an aqueous syrup formulation is reported in Figure 1.

For the sake of clarity, in order to achieve the object of the present invention, the components (or active components) (a), (b), (c) and (d) of the composition of the invention can be administered simultaneously or separately, (preferably in a time interval from 5 minutes to 30 minutes) and in any order; preferably, the active components are administered to a subject simultaneously, even more preferably in a single composition to obtain a more rapid effect and for ease of administration. When the active components of the invention are administered in a single composition, said single composition corresponds to the composition of the present invention.

In the context of the present invention, the term "subject/s" is used to indicate human or animal subjects, preferably mammals (e.g. pets such as dogs, cats, horses, sheep or cattle). Preferably, the compositions of the invention are for use in methods for the treatment of human subjects.

Unless specified otherwise, the expression composition or mixture or other comprising a component at an amount "comprised in a range from x to y" is used to indicate that said component may be present in the composition or mixture or other at all the amounts present in said range, even though not specified, extremes of the range comprised.

Unless otherwise specified, the content of a component in a composition or mixture refers to the % by weight of that component with respect to the total weight of said composition or mixture.

Unless otherwise specified, the indication that a composition or mixture or other "comprises" one or more components means that other components can be present besides that or those specifically indicated and the indication that a composition or mixture or other "consists" of determined components means that the presence of other components is excluded.

The expression "therapeutically effective amount" is used to indicate the amount of active compound and/or bacterial strain that elicits the biological or medicinal response in a tissue, system, mammal, or human being that is sought and defined by an individual, researcher, veterinarian, physician, or other clinician or health worker.

In the context of the present invention, the expression "medical device" is according to the European medical device directive [*e.g.* EU 2017/745 -(MDR), Directive 93/42/EEC-(MDD)].

EXAMPLES

Examples of the composition of the present invention in aqueous based liquid form (aqueous formulation, for example syrup) are reported in Tables 1-6.

Table 1. [a]Sucrosofate: for example, sucrose octasulfate K (anhydrous or heptahydrate)

| Substances | % w/w |
|---|---|
| Demineralised water | 60-75 |
| Magnesium Alginate | 1-5 |
| Simethicone | 0.5-5 |
| Gellan Gum | 0.1-2 |
| Sucrosofate[a] | 2.5-5 |
| Potassium Bicarbonate | 0.5-2.5 |
| Magnesium Chloride (optional) | 0.001-0.01 |
| additives and excipients | q.s. at 100 |

Table 2

| Substances | % w/w |
|---|---|
| Demineralised water | 60-75 |

(continued)

| Substances | % w/w |
|---|---|
| Magnesium Alginate | 1-5 |
| Simethicone | 0.5-5 |
| Gellan Gum | 0.1-2 |
| Sucralfate[a] | 0.1-2.5 |
| Potassium Bicarbonate | 0.5-2.5 |
| Magnesium Chloride (optional) | 0.001-0.01 |
| additives and excipients | q.s. at 100 |

Table 3

| Substances | % w/w |
|---|---|
| Demineralised water | 62-70 |
| Magnesium Alginate | 2.0-4.5 |
| Simethicone | 0.9-3.0 |
| Gellan Gum | 0.1-1.5 |
| Sucrosofate[a] | 2.5-5 |
| Potassium Bicarbonate | 1.0-2.5 |
| additives and excipients | q.s. at 100 |

Table 4

| Substances | % w/w |
|---|---|
| Demineralised water | 62-70 |
| Magnesium Alginate | 2.0-4.5 |
| Simethicone | 0.9-3.0 |
| Gellan Gum | 0.1-1.5 |
| Sucralfate[a] | 0.1-2.5 |
| Potassium Bicarbonate | 1.0-2.5 |
| additives and excipients | q.s. at 100 |

Table 5

| Substances | % w/w |
|---|---|
| Demineralised water | 67.5-74.6 |
| Magnesium Alginate | 0.5-2.5 |
| Simethicone | 1.2-4.5 |
| Gellan Gum | 0.3-1.5 |
| Sucrosofate[a] | 2.5-5 |
| Potassium Bicarbonate | 0.7-1.2 |
| Magnesium Chloride | 0.005-0.01 |
| additives and excipients | q.s. at 100 |

Table 6

| Substances | % w/w |
| --- | --- |
| Demineralised water | 67.5-74.6 |
| Magnesium Alginate | 0.5-2.5 |
| Simethicone | 1.2-4.5 |
| Gellan Gum | 0.3-1.5 |
| Sucralfate[a] | 0.1-2.5 |
| Potassium Bicarbonate | 0.7-1.2 |
| Magnesium Chloride | 0.005-0.01 |
| additives and excipients | q.s. at 100 |

EXPERIMENTAL PART

(I) FORMATION of the floating raft

(I.A) Formation and permanence of the floating raft in an acidic environment of a composition comprising magnesium alginate and gellan gum.

1. Materials and instruments:

- 0.1 M hydrochloric acid solution;
- pHenomenal electrode pH meter (VWR);
- product under analysis (see point 2.).

2. Preparation of the product under analysis

Using the preparation process of the invention, 300 g of composition according to the invention (hereinafter, product) containing magnesium alginate and gellan gum having the formulation described in Table 7 were prepared.

Table 7

| Substances | % w/w |
| --- | --- |
| Demineralised water | 67.0-70.5 |
| Magnesium Alginate | 1.5-3.1 |
| Simethicone | 0.8-2.0 |
| Gellan Gum | 0.1-0.6 |
| Potassium hydroxide | 0.05-0.4 |
| Potassium Bicarbonate | 0.5-1.2 |
| Magnesium Chloride | 0.005-0.01 |
| additives and excipients | q.s. at 100 |

3. Formation of the floating raft

In order to determine the formation of the floating raft, about 50 ml of 0.1 M hydrochloric acid solution were collected and transferred to a 50 ml Erlenmeyer flask. Using a common measuring spoon with a capacity of about 15 g, the product containing magnesium alginate and gellan gum was poured into the acidic solution.
The content of the Erlenmeyer flask was observed for 24 hours without moving or stirring it.

4. Results and discussion

4.1 Measuring the pH

Using an electrode pH meter, the recorded pH of the 0.1 M hydrochloric acid solution was found to be equal to 1.02.

4.2. Observing the floating raft

It was observed that the product containing magnesium alginate and gellan gum is capable of forming a gel immediately after contact with the acidic solution. After a short while, this gel tends to float positioning itself on the surface of the solution; as shown in Figure 2. After 24 hours, taking care not to move the system at all and avoiding any perturbation, the presence of the gel formed on the surface of the solution was still observed.

5. Conclusions

Based on the observations made, it can be stated that the product under examination (composition according to the invention), containing magnesium alginate and gellan gum, in contact with an acidic solution at pH 1.02, is capable of forming a gel capable of floating on the solution; this gel is capable of remaining for at least 24 hours.

(I.B) Comparison in the formation and permanence of the floating raft in acidic environment of a composition comprising gellan gum and of a composition comprising xanthan gum.

Study design

The ability to form the floating raft was tested *in vitro* by contacting with hydrochloric acid (0.1 M):

- a gellan gum suspension (in short, sGG), or
- a xanthan gum suspension (in short, sGX).

Products under analysis

Preparation of the polymeric suspensions:

- Gellan gum suspension (sGG): 0.30 g of gellan gum were transferred to a beaker containing 99.70 g of demineralised water. The system was placed under magnetic stirring until it had a homogeneous appearance, obtaining a gellan gum suspension in water at a concentration of 0.3% w/w;
- Xanthan gum suspension (sGX): using a process similar to the one described for sGG, a xanthan gum suspension in water at a concentration of 0.25% w/w (0.25 g of xanthan gum and 99,75 g of demineralised water) was obtained.

A small amount of erythrosine was added to both suspensions in order to make the suspensions coloured and to evaluate gel formation more efficiently.

Method

In order to determine the gelling ability of the polysaccharides under examination (sGG and sGX), two aliquots of a 0.1 M hydrochloric acid solution of about 40 ml each were collected and transferred into two 50 ml Erlenmeyer flasks. Approximately 15 ml of sGG and 15 ml of sGX were poured into each Erlenmeyer flask, respectively. Using an electrode pH meter, the recorded pH of the 0.1 M hydrochloric acid solution was found to be equal to 1.02.

The gelling ability was evaluated through a visual analysis of the two obtained systems; furthermore, each aliquot was filtered using paper-filter; lastly, both the filtrates and filters were observed.

Results

After pouring the two polysaccharide suspensions (sGG and sGX) into the respective Erlenmeyer flasks containing the 0.1 M hydrochloric acid solution, it was observed that the gellan gum immediately forms a compact gel which floats in the solution while the Xanthan Gum is fully dispersed (figures omitted). The two acidic solutions were subsequently filtered with paper-filter so as to determine the actual gel formation. With regard to gellan gum on the paper-filter, a compact gel was collected and the filtrate was found to be entirely colourless, indicating that the instantaneous gelling of the polysaccharide did not allow the diffusion of the colouring molecule in the acidic solution (Figures 3a and 3b right). The xanthan gum paper-filter, on the other hand, did not retain anything and the filtered solution is coloured, given that no gel was formed and the colouring molecule is diffused in the acidic solution (Figures 3a and 3b left).

Said results show that, unlike xanthan gum, gellan gum has the gelling ability in an acidic environment. In the light of the above, it is clear that the raft resulting from the combined and/or synergistic action of magnesium alginate/gellan gum is more compact and long-lasting with respect to a combination of magnesium alginate/xanthan gum.

(II). MUCOADHESIVE CAPACITY

The mucoadhesive capacity of a substance is closely related to the interaction of the substance under examination with the mucins present on the outer layer of the mucosa of interest (e.g. oesophageal mucosa).

Based on this principle, in order to evaluate the functional differences in the use of gellan gum with respect to xanthan gum in formulations containing magnesium alginate, there was evaluated the mucoadhesiveness of

- a formulation 1 comprising magnesium alginate and gellan gum and
- a formulation 2 comprising magnesium alginate and xanthan gum

by measuring the increase in viscosity in a suspension resulting from the interaction of said formulations under examination with the mucins.

1. Introduction to mucoadhesiveness and viscosity

Mucoadhesiveness can be defined as the ability of a substance or of a set of substances to adhere to a biological tissue over a prolonged period of time. There are several hypotheses that lay the theoretical basis for the explanation of this phenomenon; one of these is the theory of diffusion; which states that, in mucoadhesive polymers, this property results from the diffusion of polymer chains in the layer of glycoproteins present on the surface of the mucous membranes, and of the glycoproteins in the polymeric material; the diffusion of one layer in the other leads to the formation of semi-permanent bonds and interactions. The glycoproteins that contribute to the mucoadhesion phenomenon are referred to as mucins and they are molecules with very high molecular weight; with water, electrolytes and a small amount of fats, they form the protective mucus layer that covers the mucous membranes.

The viscosity of a suspension composed of mucins is given by the conformation of each molecule and by the intermolecular interaction forces which lead to chain entanglement. The presence of mucoadhesive polymers in the mucin suspension leads to an increase in interactions and chain entanglement such that the total viscosity of the suspension is described by the equation below (equation 1).

$$v_t = v_m + v_p + v_b$$

Equation 1

Wherein: vt = total viscosity; vm = viscosity due to the mucins; vp = viscosity due to the polymer; vb = component of the viscosity due to mucoadhesion.

Therefore, the mucoadhesive capacity of a polymer or a set of polymers can be determined by measuring the viscosities of solutions containing equal concentrations of polymers in the presence or absence of mucins (Hassen et al. "Pharmaceutical Research", 7(5), 1990; Garga et al. "Pharmaceutics", 10, 1-16, 2018).

2. Materials:

- Gellan gum (CAS No. 71010-52-1);
- Xanthan gum 200 MESH;
- Magnesium alginate (CAS No. 37251-44-8);
- Type II porcine stomach mucins (Sigma Aldrich);
- Ubbelohde viscometers.

3. Preparation of the formulations

In order to evaluate the mucoadhesiveness of the two anti-reflux syrups under examination, two different formulations (Formulations 1 and 2) containing only the polysaccharides and the pH correctors provided for in the respective products (Table 8 and 9) were prepared.

- Formulation 1: Suspension containing magnesium alginate and xanthan gum

Table 8

| Substance | % w/w |
|---|---|
| Demineralised Water | 93.00-97.80 |
| Sodium Hydroxide | 0.06-0.12 |
| Sodium Bicarbonate | 0.30-1.00 |
| Magnesium Alginate | 1.70-2.90 |
| Xanthan Gum | 0.10-0.50 |

- Formulation 2: Suspension containing magnesium alginate and gellan gum

Table 9

| Substance | % w/w |
|---|---|
| Demineralised Water | 94.00-98.50 |
| Potassium Hydrate | 0.09-0.20 |
| Sodium Bicarbonate | 0.50-1.20 |
| Magnesium Alginate | 1.90-3.10 |
| Gellan Gum | 0.15-0.55 |

4. Preparation of the of mucin suspension

Using the analytical balance, 20 g of mucins were weighed; the latter were subsequently transferred to a beaker containing 180 g of demineralised water. The system was placed under vigorous magnetic stirring for about 2 hours, in order to hydrate the mucins in an optimal manner. A mucin suspension in demineralised water at the concentration of 10% w/w was obtained using the described method.

5. Preparation of magnesium alginate suspensions

Given that different concentrations of magnesium alginate are used in the formulations under examination, the following suspensions were prepared in order to determine the contribution of the polysaccharide to mucoadhesiveness:

- Suspension 1: Magnesium Alginate 2.1% w/w;
  2.10 g of magnesium alginate (polysaccharide) were dispersed in 97.90 g of demineralised water. The system was placed under magnetic stirring until it was completely homogeneous.
- Suspension 2: Magnesium Alginate 2.3% w/w;
  Similarly, 2.30 g of magnesium alginate were dispersed in 97.70 g of demineralised water.

Four control solutions were prepared using suspensions 1 and 2:

- Control 1: Suspension 1 diluted 1:2;
  50 g of Suspension 1 were transferred to a beaker containing 50 g of demineralised water.
- Control 2: Suspension 2 diluted 1:2;
  50 g of Suspension 2 were transferred to a beaker containing 50 g of demineralised water.
- Control 3: Suspension 1 + Mucins 5 %;
  50 g of Suspension 1 were diluted 1:2 with the Mucin suspension at 10%.
- Control 4: Suspension 2 + Mucins 5 %;

50 g of Suspension 2 were diluted 1:2 with the Mucin suspension at 10%.

6. Preparation of the samples

- Sample 1: 5% w/w Mucin suspension
  50 g of the mucin suspension at 10% was diluted 1:2 by adding 50 g of demineralised water; thus obtaining a suspension of mucins at a concentration of 5% w/w.
- Sample 2: Formulation 1 diluted 1:2
  50 g of Formulation 1 (paragraph 8.1.) were transferred to a beaker containing 50 g of demineralised water.
- Sample 3: Formulation 2 diluted 1:2
  Using the same method described above, Formulation 2 was also diluted 1:2 with demineralised water.
- Sample 4: Formulation 1 + Mucins 5 % w/w
  50 g of Formulation 1 were transferred to a beaker containing 50 g of the Mucin suspension at 10% w/w
- Sample 5: Formulation 2 + Mucins 5 % w/w
  50 g of Formulation 2 were transferred to a beaker containing 50 g of Mucins 10 % w/w.

The dilutions carried out allow the same concentrations of substances under examination to be present in each sample (Table 10). After the various additions, each sample was placed under magnetic stirring for 2 hours at room temperature.

Composition of the samples under examination

Table 10

| Sample | Magnesium Alginate (% w/w) | Xanthan Gum (% w/w) | Gellan Gum (% w/w) | Mucins (% w/w) |
|---|---|---|---|---|
| Control 1 | 1.05 | - | - | - |
| Control 2 | 1.15 | - | - | - |
| Control 3 | 1.05 | - | - | 5 |
| Control 4 | 1.15 | - | - | 5 |
| Sample 1 | - | - | - | 5 |
| Sample 2 | 1.05 | 0.125 | - | - |
| Sample 3 | 1.15 | - | 0.15 | - |
| Sample 4 | 1.05 | 0.125 | - | 5 |
| Sample 5 | 1.15 | - | 0.15 | 5 |

7. Measuring viscosity and evaluating mucoadhesion

The viscosities of each sample were measured using the appropriate Ubbelohde viscometer. The viscosities were analysed at room temperature, measuring the time required for the suspensions to travel through the capillary of the viscometer, and the viscosity (v) of the sample was calculated by applying the formula described hereinafter (equation 2).

$$\text{Equation 2: } v = kt$$

wherein,

k = characteristic constant of each viscometer
t = time (expressed in seconds) required for the product to travel through the capillary.

The viscosity of three aliquots was calculated for each sample.
The evaluation of mucoadhesion was calculated using the mucoadhesion index (($\Delta$%) (equation 3)

$$\Delta\% = \frac{v_t - (v_m + v_p)}{v_m + v_p} \times 100$$

Equation 3 (F. C. Carvalho et al., "Brazilian Journal of Pharmaceutical Sciences", 46, 1-17, 2010)

wherein:

$v_t$ = mean viscosity of the system composed of polysaccharides and mucins
$v_m$= mean viscosity of the mucin suspension 5% w/w
$v_p$= mean viscosity of formulations diluted 1:2.

8. Results and discussion

8.1. Control Solutions

These solutions were analysed in order to evaluate the contribution of magnesium alginate in the interaction with mucins at the two different concentrations envisaged. The results obtained are reported in Table 11.

Table 11

| Sample | $v_1$ (cSt) | $v_2$ (cSt) | $v_3$ (cSt) | Mean | Std Dev. | I.C. |
|---|---|---|---|---|---|---|
| Sample 1 | 3.43 | 3.45 | 3.47 | 3.5 | 0.02 | 0.05 |
| Control 1 | 5.51 | 5.51 | 5.44 | 5.49 | 0.04 | 0.10 |
| Control 2 | 6.44 | 6.44 | 6.49 | 6.46 | 0.03 | 0.07 |
| Control 3 | 17.85 | 17.99 | 17.76 | 17.87 | 0.12 | 0.29 |
| Control 4 | 22.92 | 23.03 | 22.35 | 22.77 | 0.37 | 0.91 |

The following mucoadhesion indices were obtained using Equation 3 (Paragraph 7):

Suspension 1: $\Delta\%$ = 100
Suspension 2: $\Delta\%$ = 130

It is clear that a higher concentration of magnesium alginate leads to a higher mucoadhesion index; however, the difference between the two suspensions is not high.

8.2. Sample Solutions

The viscosities reported in Table 12 were calculated from the analyses carried out and from the times obtained.

Table 12

| Sample | $v_1$ (cSt) | $v_2$ (cSt) | $v_3$ (cSt) | Mean | Std Dev. | I.C. |
|---|---|---|---|---|---|---|
| Sample 1 | 3.43 | 3.45 | 3.47 | 3.5 | 0.02 | 0.05 |
| Sample 2 | 17.61 | 18.36 | 19.05 | 18.34 | 0.72 | 1.79 |
| Sample 3 | 29.33 | 30.83 | 29.11 | 29.76 | 0.94 | 2.33 |
| Sample 4 | 66.00 | 66.12 | 66.58 | 66.23 | 0.31 | 0.76 |
| Sample 5 | 163.44 | 158.4 | 151.67 | 157.84 | 5.91 | 14.67 |

In order to determine the mucoadhesiveness of xanthan gum and gellan gum, the viscosities of samples 1; 2; 4 and of samples 1; 3; 5, respectively, were compared.
From the data reported in Table 9 it is clear that both in Formulation 1 and in Formulation 2 (paragraph 3) there is a significant increase in viscosity in the presence of mucins; indicating that in both cases there are interaction phenomena between the polysaccharides and the mucins (Figure 4 and 5).

The mucoadhesion index of the two formulations was calculated based on the data obtained and using Equation 3 (paragraph 7):

- Formulation 1: Δ% = 204
- Formulation 2: Δ% = 375

The mucoadhesion index indicates that Formulation 2 (according to the present invention), comprising magnesium alginate and gellan gum, is significantly more mucoadhesive with respect to the Formulation 1 (comparative), containing magnesium alginate and xanthan gum.

9. Conclusions

The purpose of the study was to evaluate the functional differences between formulations containing xanthan gum or gellan gum combined with magnesium alginate for the treatment of gastroesophageal reflux disease and diseases or symptoms related therewith.

Given that the mucoadhesiveness of a given substance is closely related to the interaction of the substance with the mucins present on the outer layer of the mucosa (e.g. oesophageal mucosa), the increase in viscosity in the suspensions deriving from the formulations under examination in the presence of mucins was evaluated.

From the results obtained it is clear that in both the formulations (Formulation 1 and 2) there is a significant mucoadhesion capacity; as a matter fact, the viscosities obtained from the suspensions with polysaccharides and mucins are much higher than the sum of the suspensions with mucins and with polysaccharides alone (alginate + gum). Specifically, the differences observed are equal to 44.4 cSt and 125,3 cSt, respectively for Formulation 1 and Formulation 2.

Furthermore, by calculating the mucoadhesive indices, it was observed that the latter is significantly higher for Formulation 2 (according to the invention with gellan gum), with respect to Formulation 1 (comparative with xanthan gum).

It can therefore be presumed that magnesium alginate and gellan gum interact synergistically and significantly more deeply with mucins than with respect to the combination of magnesium alginate and xanthan gum. The great interaction with the mucins therefore results in a greater mucoadhesive property of the Formulation 2 (according to the invention with gellan gum); this ability would allow the relative product to adhere to the surfaces of the oesophageal mucous membranes for a longer period of time, protecting this organ from the corrosive-inflammatory action of the gastric juices backflowing during the reflux episode.

In conclusion, in the alight of the above, besides the beneficial effect of the floating raft in a gastric environment, the product containing the combination of magnesium alginate and gellan gum also shows effect on the oesophageal and/or pharyngeal mucosa, given that it is capable of forming a protective mucoadhesive layer on the mucosa.

(III). Physical-chemical and stability characteristics of a composition according to the invention

A composition according to the invention formulated in aqueous-based liquid form (i.e. syrup) was prepared using the raw materials reported in Table 4 and the process of the invention according to steps 1-8, including step 5 and implementing step 3 at 75°C.

The composition according to the invention obtained (hereinafter, product) appears as a viscous liquid, without lumps and dark pink in colour. The product is stable, showing no formation of precipitate.

The pH of the product is comprised from 8.1 to 9.0, with a density comprised from 1.063 g/ml to 1.200 g/ml. The viscosity, evaluated using a Brookfield rotational viscometer, is comprised from 1500 cps to 1550 cps (R3; 60 rpm; 90%).

From the data obtained it is clear that the product has appropriate characteristics for easy administration through the oral route.

The stability tests carried out demonstrate that the product maintains the chemical-physical and microbiological characteristics thereof for 30 months.

Experimental part

The anti-reflux compositions subject of the present invention, for example the formulations reported in Tables 1-6 were subjected to the following tests.

Reported below, by way of example, are the tests carried out on the formulation reported in Table 13. The same results and the same conclusions were also drawn for the formulations reported in the aforementioned Tables 1-6.

Product Description: ANTI-REFLUX SYRUP (Formula per 100g):

Table 13

| Components | Amount (%) |
|---|---|
| Demineralised water | |
| Potassium hydroxide | |
| Potassium Bicarbonate | |
| Methyl paraoxybenzoate | 0.123 |
| Propyl paraoxybenzoate | 0.06 |
| Sucrose octasulfate | |
| Fructose | |
| Magnesium alginate | |
| Gellan | |
| Magnesium Chloride | |
| Simethicone | |
| Erythrosine | |
| D-Panthenol | |
| Grenadine Flavour | |
| Isabella Grape Flavour | |

1. Biological evaluation to identify sensitivity effects through delayed hypersensitivity test according to ISO 10993-10:2010 ((GPMT test) ISO 10993-10:2010)). Results: the formulation of Table 13 does not give rise to sensitivity.

2. Biological evaluation to identify systemic toxicity through the systemic toxicity test according to ISO 10993-11:2017 through the oral route (systemic toxicity ISO 10993-11:2017 by the oral route). Results: the formulation of Table 13 does not cause toxic symptoms and it meets the test requirements.

3. Biological evaluation to identify the irritant effects through the oral mucosa irritation test according to ISO 10993-10:2010 (Oral mucosa irritation test ISO 10993-10:2010)). Results: the formulation of Table 13 does not irritate the oral mucosa.

4. Evaluation of cytotoxicity by means of an in vitro assay on fibroblast cell cultures - UNI EN ISO 10993-5:2009 (E). Results: The cytotoxicity assay was carried out on fibroblast cultures L929 treated with scalar concentrations (dilutions 1:2 starting from 5.0 mg/ml) of the Formulation of Table 13. Sodium dodecyl sulfate (SDS), a substance with known cytotoxic effects, was used as positive control, while an internal standard with IC50 > 0.5 mg/ml (non-cytotoxic substance) was used as a negative reference. The results obtained did not show a decrease in cell viability at the product concentrations tested, therefore IC50 > 0.5 mg/ml. A value of IC50 > 0.5 mg/ml indicates the total absence of cytotoxic effects of the product tested on fibroblast cell cultures.

**Claims**

**1.** A composition comprising:

(i) a mixture M comprising or, alternatively, consisting of:

- (a) an alginic acid or a pharmaceutical or food grade salt thereof;
- (b) a simethicone and/or dimethicone;
- (c) a gellan gum; and
- (d) sucrose octasulfate, referred to as sucrosofate, or a pharmaceutical or food grade salt thereof, or a sucrose octasulfate complex and aluminium hydroxide, referred to as sucralfate;

and, optionally, said composition comprises
(ii) at least one pharmaceutical or food grade additive and/or excipient.

2. The composition according to claim 1 wherein said mixture M comprises or, alternatively, consists of:

- (a) alginic acid or an alginate salt, preferably an alkali metal or alkaline-earth metal alginate selected from: magnesium alginate, potassium alginate, calcium alginate and a mixture thereof; more preferably magnesium alginate;
- (b) a simethicone and/or dimethicone; preferably simethicone;
- (c) gellan gum; and
- (d) sucrosofate or a salt thereof.

3. The composition according to claim 1 or 2, wherein said mixture M comprises or, alternatively, consists of:

- (a) alginic acid or a salt thereof, preferably an alkali metal or alkaline-earth metal alginate selected from the group consisting of: magnesium alginate, potassium alginate, calcium alginate and a mixture thereof; more preferably magnesium alginate;
- (b) a simethicone and/or dimethicone; preferably simethicone;
- (c) a gellan gum; and
- (d) sucralfate.

4. The composition according to any one of claims 1-3, wherein said mixture M comprises or, alternatively, consists of:

- (a) alginic acid or a salt thereof, preferably an alkali metal or alkaline-earth metal alginate selected from the group consisting of: magnesium alginate, potassium alginate, calcium alginate and a mixture thereof; more preferably magnesium alginate;
- (b) a simethicone;
- (c) a gellan gum; and
- (d) sucrosofate.

5. The composition according to any one of claims 1-4, wherein said mixture M further comprises (e) a bicarbonate and/or carbonate salt with an alkali metal or alkaline-earth metal; preferably selected from the group consisting of: potassium bicarbonate, magnesium bicarbonate, calcium bicarbonate, potassium carbonate, magnesium carbonate, calcium carbonate and mixtures thereof; more preferably potassium bicarbonate.

6. The composition according to any one of claims 1-5, wherein said mixture M further comprises (f) an alkali metal or alkaline-earth metal salt; preferably selected from the group consisting of: potassium chloride, magnesium chloride, calcium chloride and mixtures thereof; more preferably magnesium chloride.

7. The composition according to any one of claims 1-6, wherein said composition is formulated for oral use; preferably in powdered or granular solid form to be suspended or dispersed in aqueous medium or, alternatively, in liquid form for suspension or dispersion in aqueous medium; preferably of syrup.

8. The composition according to any one of claims 1-7, wherein said composition is preferably in aqueous based liquid form, preferably in form of syrup, comprises the active components in the following percentages by weight with respect to the total weight of the composition:

- water in a percentage comprised in the range from 40% to 90%; preferably from 55% to 80%, more preferably from 60% to 75%;
- said (a) alginic acid or a salt thereof, preferably magnesium alginate, in a percentage comprised in the range from 0.5% to 15%; preferably from 1% to 10%, more preferably from 1% to 5%, for example about 2.5% of magnesium alginate, preferably having a CAS No. 37251-44-8;
- preferably, said (b) if present is simethicone and/or dimethicone, preferably simethicone, in a percentage comprised in the range from 0.05% to 15%; preferably from 0.1% to 10%; more preferably from 0.5% to 5%, for example about 1.5% of simethicone, preferably having a CAS No. 8050-81-5;
- said (c) gellan gum in a percentage comprised in the range from 0.01% to 20%, preferably from 0.05% to 10%, more preferably from 0.1% to 2%, for example about 0.2% or 0.3% or 0.4% or 0.5% of gellan gum, preferably having a CAS No. 71010-52-1; and, optionally,

- said (d) bicarbonate and/or carbonate salt of an alkali metal or of an alkaline earth metal, if present, in a percentage comprised in the range from 0.1% to 10%, preferably from 0.5% to 5%, more preferably from 0.5% to 2.5%, for example about 1% of potassium bicarbonate.

9. The composition according to any one of claims 1-8, wherein in the mixture M contained in said composition, the by weight ratio between the components (a) alginate, preferably magnesium alginate, and (c) gellan gum ((a):(c)) is comprised in the range from about 1:1 to about 10:1, preferably from about 2:1 to about 8:1.

10. The composition according to any one of claims 1-9, wherein in the mixture M contained in said composition the by weight ratio between the components (a) alginate, preferably magnesium alginate, and (b) simethicone (c) gellan gum ((a):(b):(c)) is comprised in the range from about 10:10:5 to about 10:2:1, preferably from about 10:8:5 to 10:3:1, for example about 10:5.6:1.3.

11. The composition according to any one of claims 1-10, wherein said composition is for use as medicament.

12. The composition for use according to any one of claims 1-10, wherein said composition is for use in a method for the preventive and/or curative treatment of a gastric reflux, gastroesophageal reflux disease (GERD), laryngopharyngeal reflux disease (LPRD) or extraoesophageal reflux, or of diseases and/or symptoms associated with said gastric reflux, in a subject in need.

13. The composition according to any one of claims 1-10 , wherein said composition is for use in a method for the preventive and/or curative treatment of: oesophagitis or acute or chronic inflammation of the oesophagus mucosa, oesophageal ulcers, de-epithelialisation of the oesophageal mucosa, acid regurgitation, heartburn, feeling of gastric fullness, epigastric pain, dyspepsia, nausea, chronic cough, bronchospasm, inflamed throat, laryngitis, globus sensation or hypopharyngeal bolus, pyrosis, dysphonia and/or nasopharyngeal inflammation.


**Patentansprüche**

1. Zusammensetzung, umfassend:

(i) eine Mischung M, umfassend oder alternativ bestehend aus:

- (a) eine/r Alginsäure oder ein/em pharmazeutisches/en oder lebensmittelechtes/en Salz davon;
- (b) ein/em Simethicon und/oder Dimethicon;
- (c) ein/em Gellangummi; und
- (d) Saccharoseoctasulfat, das als Sucrosofat bezeichnet wird, oder ein Salz davon in pharmazeutischer oder Lebensmittelqualität oder ein Saccharoseoctasulfatkomplex und Aluminiumhydroxid, das als Sucralfat bezeichnet wird;

und optional umfasst die Zusammensetzung
(ii) mindestens einen pharmazeutischen oder lebensmittelechten Zusatzstoff und/oder Hilfsstoff.

2. Zusammensetzung nach Anspruch 1, wobei die Mischung M umfasst oder alternativ besteht aus:

- (a) Alginsäure oder ein/em Alginatsalz, vorzugsweise ein/em Alkalimetall- oder Erdalkalimetallalginat, ausgewählt aus: Magnesiumalginat, Kaliumalginat, Calciumalginat und einer Mischung davon; bevorzugter Magnesiumalginat;
- (b) ein/em Simethicon und/oder Dimethicon; vorzugsweise Simethicon;
- (c) Gellangummi; und
- (d) Sucrosofat oder ein/em Salz davon.

3. Zusammensetzung nach Anspruch 1 oder 2, wobei die Mischung M umfasst oder alternativ besteht aus:

- (a) Alginsäure oder ein/em Salz davon, vorzugsweise ein/em Alkalimetall- oder Erdalkalimetallalginat, ausgewählt aus der Gruppe bestehend aus: Magnesiumalginat, Kaliumalginat, Calciumalginat und einer Mischung davon; bevorzugter Magnesiumalginat;
- (b) ein/em Simethicon und/oder Dimethicon; vorzugsweise Simethicon;

- (c) ein/em Gellangummi; und
- (d) Sucralfat.

4. Zusammensetzung nach einem der Ansprüche 1-3, wobei die Mischung M umfasst oder alternativ besteht aus:

- (a) Alginsäure oder ein/em Salz davon, vorzugsweise ein/em Alkalimetall- oder Erdalkalimetallalginat, ausgewählt aus der Gruppe bestehend aus: Magnesiumalginat, Kaliumalginat, Calciumalginat und einer Mischung davon; bevorzugter Magnesiumalginat;
- (b) ein/em Simethicon;
- (c) ein/em Gellangummi; und
- (d) Sucrosofat.

5. Zusammensetzung nach einem der Ansprüche 1-4, wobei die Mischung M ferner (e) ein Bicarbonat- und/oder Carbonatsalz mit einem Alkalimetall oder Erdalkalimetall umfasst; vorzugsweise ausgewählt aus der Gruppe bestehend aus: Kaliumbicarbonat, Magnesiumbicarbonat, Calciumbicarbonat, Kaliumcarbonat, Magnesiumcarbonat, Calciumcarbonat und Mischungen davon; bevorzugter Kaliumbicarbonat.

6. Zusammensetzung nach einem der Ansprüche 1-5, wobei die Mischung M ferner (f) ein Alkalimetall- oder Erdalkalimetallsalz umfasst; vorzugsweise ausgewählt aus der Gruppe bestehend aus: Kaliumchlorid, Magnesiumchlorid, Calciumchlorid und Mischungen davon; bevorzugter Magnesiumchlorid.

7. Zusammensetzung nach einem der Ansprüche 1-6, wobei die Zusammensetzung zur oralen Verwendung formuliert ist; vorzugsweise in pulverisierter oder körniger fester Form, um in wässrigem Medium suspendiert oder dispergiert zu werden, oder alternativ in flüssiger Form zur Suspension oder Dispersion in wässrigem Medium; vorzugsweise von Sirup.

8. Zusammensetzung nach einem der Ansprüche 1-7, wobei die Zusammensetzung vorzugsweise in flüssiger Form auf wässriger Basis, vorzugsweise in Form von Sirup, vorliegt und die aktiven Komponenten in den folgenden Gewichtsprozenten in Bezug auf das Gesamtgewicht der Zusammensetzung umfasst:

- Wasser in einem Prozentsatz im Bereich von 40 % bis 90 %; vorzugsweise von 55 % bis 80 %, bevorzugter von 60 % bis 75 % liegt;
- die (a) Alginsäure oder ein Salz davon, vorzugsweise Magnesiumalginat, in einem Prozentsatz im Bereich von 0,5 % bis 15 %; vorzugsweise von 1 % bis 10 %, bevorzugter von 1 % bis 5 %, beispielsweise etwa 2,5 % Magnesiumalginat, vorzugsweise mit einer CAS-Nr. 37251-44-8, liegt;
- vorzugsweise ist (b), falls vorhanden, Simethicon und/oder Dimethicon, vorzugsweise Simethicon, in einem Prozentsatz im Bereich von 0,05 % bis 15 %; vorzugsweise von 0,1 % bis 10 %; bevorzugter von 0,5 % bis 5 %, beispielsweise etwa 1,5 % Simethicon, vorzugsweise mit einer CAS-Nr. 8050-81-5;
- wobei der (c) Gellangummi in einem Prozentsatz im Bereich von 0,01 % bis 20 %, vorzugsweise von 0,05 % bis 10 %, bevorzugter von 0,1 % bis 2 %, beispielsweise etwa 0,2 % oder 0,3 % oder 0,4 % oder 0,5 % Gellangummi, vorzugsweise mit einer CAS-Nr. 71010-52-1, liegt; und gegebenenfalls
- das (d) Bicarbonat und/oder Carbonatsalz eines Alkalimetalls oder eines Erdalkalimetalls, falls vorhanden, in einem Prozentsatz im Bereich von 0,1 % bis 10 %, bevorzugt von 0,5 % bis 5 %, bevorzugter von 0,5 % bis 2,5 %, beispielsweise etwa 1 % Kaliumbicarbonat liegt.

9. Zusammensetzung nach einem der Ansprüche 1-8, wobei in der in der Zusammensetzung enthaltenen Mischung M das Gewichtsverhältnis zwischen den Komponenten (a) Alginat, vorzugsweise Magnesiumalginat, und (c) Gellangummi ((a):(c)) im Bereich von etwa 1:1 bis etwa 10:1, vorzugsweise von etwa 2:1 bis etwa 8:1, liegt.

10. Zusammensetzung nach einem der Ansprüche 1-9, wobei in der in der Zusammensetzung enthaltenen Mischung M das Gewichtsverhältnis zwischen den Komponenten (a) Alginat, vorzugsweise Magnesiumalginat, und (b) Simethicon (c) Gellangummi ((a): (b):(c)) im Bereich von etwa 10:10:5 bis etwa 10: 2:1, vorzugsweise von etwa 10:8:5 bis 10:3:1, beispielsweise etwa 10:5,6:1,3, liegt.

11. Zusammensetzung nach einem der Ansprüche 1-10, wobei die Zusammensetzung zur Verwendung als Medikament bestimmt ist.

12. Zusammensetzung zur Verwendung nach einem der Ansprüche 1-10, wobei die Zusammensetzung zur Verwendung in einem Verfahren zur präventiven und/oder kurativen Behandlung eines Magenreflux, einer gastroösophagealen Refluxkrankheit (GERD), einer laryngopharyngealen Refluxkrankheit (LPRD) oder eines extraösophagealen Reflux oder von Krankheiten und/oder Symptomen, die mit dem Magenreflux assoziiert sind, bei einem bedürftigen Patienten ist.

13. Zusammensetzung nach einem der Ansprüche 1-10, wobei die Zusammensetzung zur Verwendung in einem Verfahren zur präventiven und/oder kurativen Behandlung von: Ösophagitis oder akuter oder chronischer Entzündung der Ösophagusschleimhaut, Ösophagusgeschwüren, Deepithelialisierung der Ösophagusschleimhaut, saurem Aufstoßen, Sodbrennen, Gefühl von Magenfülle, epigastrischem Schmerz, Dyspepsie, Übelkeit, chronischem Husten, Bronchospasmus, entzündetem Hals, Laryngitis, Globusempfindung oder hypopharyngealem Bolus, Pyrose, Dysphonie und/oder nasopharyngealer Entzündung bestimmt ist.

**Revendications**

1. Composition, comprenant :

   (i) un mélange M comprenant ou, alternativement, constitué :

   - (a) d'un acide alginique ou d'un sel pharmaceutique ou de qualité alimentaire de celui-ci ;
   - (b) d'un siméthicone et/ou d'un diméthicone ;
   - (c) d'une gomme gellane ; et
   - (d) de octasulfate de saccharose, appelé sucrosofate, ou un sel pharmaceutique ou de qualité alimentaire de celui-ci, ou d'un complexe d'octasulfate de saccharose et d'hydroxyde d'aluminium, appelé sucralfate ;

   et, éventuellement, ladite composition comprend
   (ii) au moins un additif et/ou excipient pharmaceutique ou de qualité alimentaire.

2. Composition selon la revendication 1, dans laquelle ledit mélange M comprend ou, alternativement, est constitué :

   - (a) d'acide alginique ou d'un sel d'alginate, de préférence un alginate de métal alcalin ou alcalino-terreux choisi parmi : l'alginate de magnésium, l'alginate de potassium, l'alginate de calcium et un mélange de ceux-ci ; plus préférablement d'alginate de magnésium ;
   - (b) d'un siméthicone et/ou d'un diméthicone ; de préférence le siméthicone ;
   - (c) de gomme gellane ; et
   - (d) de sucrosofate ou un sel de celui-ci.

3. Composition selon la revendication 1 ou 2, dans laquelle ledit mélange M comprend ou, alternativement, est constitué :

   - (a) d'acide alginique ou d'un sel de celui-ci, de préférence un alginate de métal alcalin ou alcalino-terreux choisi dans le groupe constitué par : l'alginate de magnésium, l'alginate de potassium, l'alginate de calcium et un mélange de ceux-ci ; plus préférablement l'alginate de magnésium ;
   - (b) d'un siméthicone et/ou d'un diméthicone ; de préférence le siméthicone ;
   - (c) d'une gomme gellane ; et
   - (d) de sucralfate.

4. Composition selon l'une quelconque des revendications 1-3, dans laquelle ledit mélange M comprend ou, alternativement, est constitué :

   - (a) d'acide alginique ou d'un sel de celui-ci, de préférence un alginate de métal alcalin ou alcalino-terreux choisi dans le groupe constitué par : l'alginate de magnésium, l'alginate de potassium, l'alginate de calcium et un mélange de ceux-ci ; plus préférablement l'alginate de magnésium ;
   - (b) d'un siméthicone ;
   - (c) d'une gomme gellane ; et
   - (d) de sucrosofate.

5. Composition selon l'une quelconque des revendications 1-4, dans laquelle ledit mélange M comprend en outre (e) un sel de bicarbonate et/ou de carbonate avec un métal alcalin ou un métal alcalino-terreux ; de préférence choisi dans le groupe constitué par : le bicarbonate de potassium, le bicarbonate de magnésium, le bicarbonate de calcium, le carbonate de potassium, le carbonate de magnésium, le carbonate de calcium et leurs mélanges ; plus préférablement le bicarbonate de potassium.

6.  Composition selon l'une quelconque des revendications 1-5, dans laquelle ledit mélange M comprend en outre (f) un sel de métal alcalin ou alcalino-terreux ; de préférence choisi dans le groupe constitué par : le chlorure de potassium, le chlorure de magnésium, le chlorure de calcium et leurs mélanges ; plus préférablement le chlorure de magnésium.

7.  Composition selon l'une quelconque des revendications 1-6, dans laquelle ladite composition est formulée pour une utilisation orale ; de préférence sous forme solide en poudre ou granulaire à mettre en suspension ou à disperser dans un milieu aqueux ou, alternativement, sous forme liquide pour une mise en suspension ou une dispersion dans un milieu aqueux ; de préférence sous forme de sirop.

8.  Composition selon l'une quelconque des revendications 1-7, dans laquelle ladite composition se présente de préférence sous forme liquide à base aqueuse, de préférence sous forme de sirop, comprend les composants actifs dans les pourcentages en poids suivants par rapport au poids total de la composition :

    - de l'eau dans un pourcentage compris de 40 à 90 % ; de préférence de 55 à 80 %, plus préférablement de 60 à 75 % ;
    - ledit (a) acide alginique ou un sel de celui-ci, de préférence l'alginate de magnésium, dans un pourcentage compris de 0,5 à 15 % ; de préférence compris de 1 à 10 %, plus préférablement compris de 1 à 5 %, par exemple environ 2,5 % d'alginate de magnésium, de préférence ayant un numéro CAS 37251-44-8 ;
    - de préférence, ledit (b), s'il est présent, est du siméthicone et/ou du diméthicone, de préférence du siméthicone, dans un pourcentage compris de 0,05 à 15 % ; de préférence de 0,1 à 10 % ; plus préférablement de 0,5 à 5 %, par exemple environ 1,5 % de siméthicone, de préférence ayant le numéro CAS 8050-81-5 ;
    - ladite (c) gomme gellane dans un pourcentage compris de 0,01 à 20 %, de préférence de 0,05 à 10 %, plus préférablement de 0,1 à 2 %, par exemple environ 0,2 % ou 0,3 % ou 0,4 % ou 0,5 % de gomme gellane, de préférence ayant un numéro CAS 71010-52-1 ; et, éventuellement,
    - ledit (d) bicarbonate et/ou sel de carbonate d'un métal alcalin ou d'un métal alcalino-terreux, s'il est présent, dans un pourcentage compris de 0,1 à 10 %, de préférence de 0,5 à 5 %, plus préférablement de 0,5 à 2,5 %, par exemple environ 1 % de bicarbonate de potassium.

9.  Composition selon l'une quelconque des revendications 1-8, dans laquelle, dans le mélange M contenu dans ladite composition, le rapport pondéral entre les composants (a) alginate, de préférence l'alginate de magnésium, et (c) la gomme gellane ((a):(c)) est compris d'environ 1:1 à environ 10:1, de préférence d'environ 2:1 à environ 8:1.

10. Composition selon l'une quelconque des revendications 1-9, dans laquelle, dans le mélange M contenu dans ladite composition, le rapport pondéral entre les composants (a) alginate, de préférence l'alginate de magnésium, et (b) le siméthicone et (c) la gomme gellane ((a) : (b) : (c)) est compris d'environ 10:10:5 à environ 10:2:1, de préférence d'environ 10:8:5 à 10:3:1, par exemple environ 10:5.6:1.3.

11. Composition selon l'une quelconque des revendications 1-10, dans laquelle ladite composition est destinée à être utilisée comme médicament.

12. Composition destinée à être utilisée selon l'une quelconque des revendications 1-10, dans laquelle ladite composition est destinée à être utilisée dans un procédé de traitement préventif et/ou curatif d'un reflux gastrique, de la maladie du reflux gastro-œsophagien (RGO), de la maladie du reflux laryngopharyngien (RLP) ou du reflux extra-œsophagien, ou des maladies et/ou symptômes associés audit reflux gastrique, chez un sujet qui en a besoin.

13. Composition selon l'une quelconque des revendications 1-10, dans laquelle ladite composition est destinée à être utilisée dans un procédé de traitement préventif et/ou curatif de l'œsophagite ou de l'inflammation aiguë ou chronique de la muqueuse œsophagienne, des ulcères œsophagiens, de la désépithélialisation de la muqueuse œsophagienne, de la régurgitation acide, des brûlures d'estomac, de la sensation de plénitude gastrique, de douleurs épigastriques, de la dyspepsie, des nausées, de la toux chronique, des bronchospasmes, de l'inflammation de la gorge, de la laryngite, de la sensation de boule hystérique ou de la boule de l'hypopharynx, de pyrosis, de dysphonie et/ou d'inflammation nasopharyngienne.

Demineralised water

Stock Aliquot

Aliquot 1
(35% with respect to the amount of total demineralised water)

Add according to the indicated order

Heat to 75°

1. Preservatives
2. Excipients
3. Magnesium alginate
4. Simethicone
5. dye
6. flavour

Add: Gellan Gum

Add: Magnesium Chloride

Keep under stirring at 75°

Combine the two aliquots and stir

Stock Aliquot + Aliquot 1

Product

Allow to cool at room temperature

Stir again and break the gel formed by cooling

Final Product

Figure 1

Figure 2

Gellan Filter

Xanthan Filter

Figure 3a

Gellan
Gum
0.3% w/w
Filtrate

Gellan
Gum
0.25%
Filtrate

Figure 3b

FORMULATION 1

Figure 4

FORMULATION 2

Figure 5

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- GB 2349570 A

## Non-patent literature cited in the description

- *CHEMICAL ABSTRACTS*, 37251-44-8
- *CHEMICAL ABSTRACTS*, 9005-32-7
- *CHEMICAL ABSTRACTS*, 90005-35-0
- *CHEMICAL ABSTRACTS*, 90005-36-1
- *CHEMICAL ABSTRACTS*, 90005-38-3
- *CHEMICAL ABSTRACTS*, 8050-81-5
- *CHEMICAL ABSTRACTS*, 63148-62-9
- *CHEMICAL ABSTRACTS*, 68937-54-2
- *CHEMICAL ABSTRACTS*, 68554-53-0
- *CHEMICAL ABSTRACTS*, 71010-52-1
- *CHEMICAL ABSTRACTS*, 11138-66-2
- *CHEMICAL ABSTRACTS*, 9000-30-0
- *CHEMICAL ABSTRACTS*, 9004-61-9
- *CHEMICAL ABSTRACTS*, 76578-81-9
- *CHEMICAL ABSTRACTS*, 74135-10-7
- *CHEMICAL ABSTRACTS*, 57680-56-5
- *CHEMICAL ABSTRACTS*, 54182-58-0
- **HASSEN et al.** *Pharmaceutical Research*, 1990, vol. 7 (5)
- **GARGA et al.** *Pharmaceutics*, 2018, vol. 10, 1-16